# EUROPEAN PATENT SPECIFICATION

(11) **EP 1 559 327 B1**
(45) Date of publication and mention of the grant of the patent: **12.05.2010**
(21) Application number: 04030873.6
(22) Date of filing: 22.03.2002
(51) Int. Cl.: A23K 1/00, A23K 1/18

(54) **Process for the preparation of a bacterial feed for aquaculture and its use**
Verfahren zur Herstellung eines Futtermittels enthaltend Bakterien für Aquakultur und seiner Verwendung
Procédé de préparation d'un aliment comprenant des microbes pour l'aquaculture et son utilisation

(30) Priority: 23.03.2001 US 277947 P
(43) Date of publication of application: 03.08.2005
(62) Divisional of application: 02725276.6
(73) Proprietor: Advanced Bionutrition Corporation, Columbia, MD 21045 (US)
(72) Inventor: Kyle, David.J., Catonsville, MD 21228 (US)
(74) Representative: Warcoin, Jacques

(56) References cited:
- EP-A- 0 954 978
- EP-A- 0 971 034
- GB-A- 2 316 082
- LEONG J C ET AL: "FISH VACCINE ANTIGENS PRODUCED OR DELIVERED BY RECOMBINANT DNA TECHNOLOGIES" DEVELOPMENTS IN BIOLOGICAL STANDARDIZATION, KARGER, BASEL, CH, vol. 90, 1997, pages 267-277, XP001062656 ISSN: 0301-5149

## Description

### BACKGROUND OF THE INVENTION

### Field of the Invention

This invention is directed to the use of probiotic bacteria which are used as feed components in aquaculture, and which also contain exogenous peptides and/or antibodies, which will convey resistance or immunity to viral or bacterial pathogens or otherwise improve the health and performance of the species consuming said probiotic bacteria. The peptides and/or antibodies are expressed inside the probiotic bacteria by direct genetic modification of the probiotic bacteria itself, or by the infection of the probiotic bacteria with a virus that has been altered to express the peptide and/or antibody of interest.

### Related Art

Certain plant products have been produced using specific genetic modification to express proteins and/or antibodies of therapeutic value. The group at the Boyce Thompson Institute at Cornell has been cloning viral coat protein into bananas and potatoes so that when ingested, this will be equivalent to delivering an oral vaccine. This concept has not been extended to microbes.

There are several plant biotech companies such as Meristem, Large Scale Biology, and Prodigene, which are now expressing certain human therapeutic proteins in the plants including antibodies.

Recombinant microbes including bacteria, yeast and fungi have been used to produce human therapeutic proteins. However, such recombinant microbes have not been used for agricultural purposes incorporating ingestion of the whole organism. In both the plant and microbial cases, the recombinant organism has simply been used as a factory, and the therapeutic protein is then isolated and purified prior to use.
LEONG et al (DEVELOPMENTS IN BIOLOGICAL STANDARDIZATION, vol.90, 1997, pages 267-277) discloses aquaculture vaccine delivery by recombinant DNA technology, corresponding to immunogenic proteins, and employing a bacterium as a host system.
US 6,100,388 discloses the use of a modified *Lactobacillus reuteri* to express an immunogenic agent. Finally, none of these documents discloses the use of probiotic bacterial biomass for expressing antibodies and/or bactericidal and bacteriostatic peptides.

Certain plant products have been produced which contain proteins and/or antibodies of therapeutic value by infecting the plant with a virus that expresses the protein of interest. Large Scale Biology has a series of patents protecting this technology but these patents do not disclose the use of the technology in microbes.

Antibiotic doping is used routinely in the aquaculture setting. Typically, the pure or semipure antibiotics are added directly to the water column; however, crude fermentation broths, or crude preparations including cells, have not been used for any kind of therapeutic delivery system.

Production of amino acids such as lysine typically involves a genetically modified microorganism, which overproduces the amino acid of interest and excretes it into the fermentation medium. The wastestream from such fermentation would include biomass containing the amino acid, and this wastestream product could be used as a crude delivery form of the small molecule nutritive amino acid.

### SUMMARY OF THE INVENTION

The present invention provides for a process for the production of a composition of matter (the feed) and the use of this feed for the preparation of a medicament.

In one embodiement, this invention provides a process for the production of a feed composition for shellfish in aquaculture comprising the steps of:
a) transforming a probiotic bacterial biomass with a virus or a plasmid, which express:
   (i) bactericidal and bacteriostatic peptides selected from the group comprising from cecropins, penaedins, bactenecins, callinectins, myticins, tachyplesins, clavanins, misgurins, pleurocidins, parasins, histones, acidic proteins, and lysozymes which are non-native to said bacterial biomass; or
   (ii) antibodies;
b) culturing the probiotic bacterial biomass obtained in step a) to produce said bactericidal and bacteriostatic peptides or antibodies; and
c) formulating the probiotic bacterial biomass obtained in step b) as a feed composition.

In another embodiment, this invention provides a method of preparing a feed for delivering therapeutic proteins to a shellfish, said feed comprising a probiotic bacterium expressing a non-native therapeutic peptides. In a preferred mode, the therapeutic protein is a recombinant protein expressed by the probiotic bacterium or the probiotic bacterium is infected by a recombinant virus, which expresses the recombinant therapeutic or bioactive protein. Preferred therapeutic proteins include a protein which inhibits growth or replication of Vibrio species in vitro, or a protein which inhibits Taura Syndrome Virus (TSV) or White Spot Syndrome Virus (WSSV) infection in shrimp, or a recombinantly expressed antibody.

### DETAILED DESCRIPTION OF THE EMBODIMENTS

Yeast, fungi and bacteria are in the direct food chain of fish, crustaceans and molluscs. However, only a very few of these microbes, perhaps less than 10 species, have been exploited for aquaculture feeds. These few species have been used primarily for historical reasons and ease of cultivation. They have not been chosen on the basis of any scientific evidence of superiority as nutritional or therapeutic supplements.

The marine environment is filled with bacteria and viruses that can attack fish and shellfish thereby devastating intensive farms very quickly. Bacteria and viruses can also attack single celled microalgae, so these organisms have evolved biochemical mechanisms to defend themselves from such attacks. Such mechanisms may involve the secretion of compounds that inhibit bacterial growth or viral attachment. Such compounds are called "prebiotics" and have effects similar to how cranberry juice can prevent bladder infections in humans. When nutritional, therapeutic or protective effects are delivered via the whole live organisms, such as *Lactobacillis* in yogurt, such products are referred to as "probiotics" and the organisms are "probionts". For the purposes of this invention, both types of action will be referred to as probiotic.

The main aspect of this invention is directed to the use of recombinant probiotic bacteria or virus infected probiotic bacteria to deliver the bioactive protein of choice. The recombinant probiotic bacteria or virus-infected probiotic bacteria may be tested for antibiotic activity by standard antibiotic screening assays to confirm their activity

One of the major disease control problems in shrimp aquaculture today is infection by certain viruses (e.g., White Spot Syndrome Virus and Tara Syndrome Virus). Neither current antibiotic, nor probiotic strategies will work in this situation, and shrimp cannot be vaccinated in a way similar to fish. Shrimp have only a rudimentary immune system so they are particularly susceptible to devastation by viral attacks. This invention provides a solution to this problem using a biological control method using probiotic bacteria as the vector to deliver anti-White Spot antibodies directly to the shrimp.
These "Designer feeds" would be a normal part of the diet, but modified to deliver a therapeutic dose of antibody directly to the gut of the shrimp. This approach is known as "passive immunity" because the antibody remains outside the host organism and simply prevents infestation through the gut wall. The invention envisions the use of probiotic bacteria to deliver the antibody to the virus. Such probiotics, as envisioned in the invention, do not have to replicate in the target organism for the desired effect to occur. Alternatively, the probiotic bacterium itself may be infected with a virus that is engineered to produce the antibody of interest.

Antibodies to desired targets, such as White Spot Syndrome Virus or Taura Syndrome Virus, may be prepared by routine immunization and selection of monoclonal antibody producing hybridomas, or by screening viral or bacterial expression libraries of immunoglobulin genes and gene fragments. See "Current Protocols in Immunology," Coligan, et al., eds, Wiley Interscience, 1991, and periodic supplements. Nucleic acid sequences encoding the binding sites of the selected antibodies can be cloned using standard methods (see "Current Protocols in Molecular Biology." Ausubel, et al., eds., Wiley-Interscience, 1987, and periodic supplements), and antibodies may be expressed from recombinant probiotic bacteria or cloned into viruses that infect the desired probiotic bacteria.

There are a number of bactericidal and bacteriostatic peptides, which will inhibit microbial growth and that include, but are not limited to cecropins, penaeidins, bactenecins, callinectins, myticins, tachyplesins, clavanins, misgurins, pleurocidins, parasins, histones, acidic proteins, and lysozymes. These peptides may be expressed in a microbial biomass such as algae, yeast, fungi or bacteria using recombinant methods as described above, and thus provided as a feed component to convey resistance to infestation.

The invention as contemplated herein, is described in the following examples, but its utility is not limited to the examples provided.

### EXAMPLES

**Example 1. Expression of a bactericidal protein in a microbial feed.** A bactericidal protein is chosen for the particular application. For example, proteins of the penaeidin class may be chosen for pathogenic control in shrimp. Penaeidins are members of a family of antimicrobial peptides isolated from crustaceans (e.g., Penaeid shrimp).
Antimicrobial peptides may also come from insects and chelicerates and may include but are not limited to cecropins, peneaidins, bactenecins, callinectins, myticins, tachyplesins, clavanins, misgunins, pleurocidins, parasins, histones, acidic proteins, and lysozymes. The gene for the chosen protein or peptide is either isolated from the original source, an amplification source, or it can be made synthetically. The gene is then incorporated into a transformation vector suitable for a eukaryotic algae (e.g. Chlorella) or a prokaryotic alga (e.g. *Synechocystis*)*,* or a yeast (e.g. *Saccharomyces*) or a fungus (e.g. *Mortierella*). The transformation vector is chosen so that the protein will be over expressed in the microbial cell biomass. This biomass is then used as a feed additive in such a way as to provide the bactericidal protein directly to the animal thus providing resistance to that particular pathogen.

**Example 2. Delivery of active peptides or proteins using probiotic feeds.**
The gene for an active antimicrobial peptide, such as, but not limited to, cecropins, peneaidins, bactenecins, callinectins, myticins, tachyplesins, clavanins, misgurins, pleurocidins, or parasins, or an antimicrobial protein such as histones, acidic proteins, or lysozymes is isolated and inserted into a transformation vector selected for use in Lactobacillus using molecular biology methods that are well know in the state of the art.
The recombinant Lactobacillus cells expressing the free peptides or proteins, or excreted proteins, are then grown using conventional fermentation technology, harvested and processed into a form usable as a feed for an animal such as, but not limited to fish, crustaceans, livestock, etc. This form may include, but is not limited to freeze drying, spray drying, fluid bed drying, microencapsulation, extrusion, or tableting. The recombinant Lactobacillus is then provided to the animal as a feed, thereby delivering both the valuable probiotic as well as the antimicrobial compound. In this case, the antimicrobial compound is constantly produced as long as the recombinant Lactobacillus is present in the gut of the animal.

## Claims

1. A process for the production of a feed composition for shellfish in aquaculture comprising the steps of:
a) transforming a probiotic bacterial biomass with a virus or a plasmid, which express:
(i) bactericidal and bacteriostatic peptides selected from the group comprising from cecropins, penaedins, bactenecins, callinectins, myticins, tachyplesins, clavanins, misgurins, pleurocidins, parasins, histones, acidic proteins, and lysozymes which are non-native to said bacterial biomass; or
(ii) antibodies;
b) culturing the probiotic bacterial biomass obtained in step a) to produce said bactericidal and bacteriostatic peptides or antibodies; and
c) formulating the probiotic bacterial biomass obtained in step b) as a feed composition.

2. The process according to claim 1, wherein the probiotic bacteria is infected by a recombinant virus, which expresses said one or more bacterial and bacteriostatic peptides, or antibodies.

3. The process according to claim 1, for the production of a feed composition comprising a bacterial biomass comprising antibodies.

4. The process according to claim 1 for shrimp aquaculture.

5. The process according to claim 4, wherein said antibodies target White Spot Syndrome virus or Taura Syndrome virus.

6. The process according to claim 1, for the production of a feed composition comprising a bacterial biomass comprising bactericidal and bacteriostatic peptides selected from the group comprising from cecropins, penaedins, bactenecins, callinectins, myticins, tachyplesins, clavanins, misgurins, pleurocidins, parasins, histones, acidic proteins, and lysozymes which are non-native to said bacterial biomass.

7. The process according to claim 6, wherein the bacteria is lactobacillus.

8. The process according to claim 7, wherein the probiotic bacteria is selected from the group comprising Lactobacillus, Bacillus and Bifidobacterium.

9. The process according to claim 1, wherein the step c) corresponds to freeze drying, spray drying, fluid bed drying, microencapsulation, extrusion or tabletting.

10. Use in aquaculture of a feed composition obtainable by the process according to claim 5 for the preparation of a medicament which inhibits Taura Syndrome Virus (TSV) or White Spot Syndrome Virus (WSSV) infection in shrimp.

11. Use in aquaculture of a feed composition obtainable by the process according to claim 6 for the preparation of a medicament that conveys resistance to infestation.

## Patentansprüche

1. Verfahren zur Herstellung eines Futtermittels für Schalentiere in Aquakultur, welches die folgenden Schritte umfasst:
(a) Infizieren einer probiotischen bakteriellen Biomasse mit einem Virus oder einem Plasmid, welche exprimiert:
(i) bakterizide und bakteriostatische Peptide, die ausgewählt sind aus der Gruppe umfassend Cecropine, Penaedine, Bactenecine, Callinectine, Myticine, Tachyplesine, Clavanine, Misgurine, Pleurocidine, Parasine, Histone, saure Proteine und Lysozyme, die für die bakterielle Biomasse nicht-nativ sind; oder
(ii) Antikörper;
(b) Kultivieren der in Schritt (a) erhaltenen probiotischen bakteriellen Biomasse, um die bakteriziden und bakteriostatischen Peptide oder Antikörper zu produzieren; und
(c) Formulieren der in Schritt (b) erhaltenen probiotischen Biomasse als Futtermittel.

2. Verfahren nach Anspruch 1, bei dem die probiotischen Bakterien mit einem rekombinanten Virus infiziert werden, der ein oder mehrere bakterizide und bakteriostatische Peptide oder Antikörper exprimiert.

3. Verfahren nach Anspruch 1 für die Produktion eines Futtermittels, das eine bakterielle Biomasse umfasst, welche Antikörper umfasst.

4. Verfahren nach Anspruch 1 für eine Krevette-Aquakultur.

5. Verfahren nach Anspruch 4, bei dem die Antikörper auf das White-Spot-Syndrom-Virus oder Taura-Syndrom-Virus abzielen.

6. Verfahren nach Anspruch 1 zur Produktion eines Futtermittels, das eine bakterielle Biomasse umfasst, welche bakterizide oder bakteriostatische Peptide umfasst, die ausgewählt sind aus der Gruppe umfassend Cecropine, Penaedine, Bactenecine, Callinectine, Myticine, Tachyplesine, Clavanine, Misgurine, Pleurocidine, Parasine, Histone, saure Proteine und Lysozyme, die für die bakterielle Biomasse nicht-nativ sind.

7. Verfahren nach Anspruch 6, bei dem das Bakterium Lactobacillus ist.

8. Verfahren nach Anspruch 7, bei dem das probiotische Bakterium ausgewählt ist aus der Gruppe umfassend Lactobacillus, Bacillus und Bifidobacterium.

9. Verfahren nach Anspruch 1, bei dem der Schritt c) einer Gefriertrocknung, Sprühtrocknung, Fließbett-trocknung, Mikroverkapselung, Extrusion oder Tablettierung entspricht.

10. Verwendung eines Futtermittels in Aquakultur, das durch das Verfahren nach Anspruch 5 erhältlich ist, für die Herstellung eines Medikaments, das eine Taura-Syndrom-Virus (TSV)- oder eine White-Spot-Syndrom-Virus (WSSV)-Infektion bei Krevette hemmt.

11. Verwendung eines Futtermittels in Aquakultur, das durch das Verfahren nach Anspruch 6 erhältlich ist, für die Herstellung eines Medikaments, das eine Resistenz gegen den Befall vermittelt.

## Revendications

1. Procédé de production d'une composition nutritive pour crustacés en aquaculture comprenant les étapes consistant à :
a) transformer une biomasse bactérienne probiotique avec un virus ou un plasmide, laquelle exprime :
(i) des peptides bactéricides et bactériostatiques choisis dans le groupe comprenant les cécropines, les pénédines, les bacténécines, les callinectines, les myticines, les tachyplésines, les clavanines, les misgurines, les pleurocidines, les parasines, les histones, les protéines acides et les lysozymes non natifs vis-à-vis de ladite biomasse bactérienne ; ou
(ii) des anticorps ;
b) cultiver la biomasse bactérienne probiotique obtenue à l'étape a) pour produire lesdits peptides bactéricides et bactériostatiques ou anticorps ; et
c) formuler la biomasse bactérienne probiotique obtenue à l'étape b) sous la forme d'une composition nutritive.

2. Procédé selon la revendication 1, dans lequel la bactérie probiotique est infectée par un virus recombinant qui exprime ledit ou lesdits peptides bactéricides et bactériostatiques ou anticorps.

3. Procédé selon la revendication 1, pour la production d'une composition nutritive comprenant une biomasse bactérienne comprenant des anticorps.

4. Procédé selon la revendication 1 pour l'aquaculture de crevettes.

5. Procédé selon la revendication 4, dans lequel lesdits anticorps ciblent le virus du syndrome des taches blanches ou le virus du syndrome de Taura.

6. Procédé selon la revendication 1 pour la production d'une composition nutritive comprenant une biomasse bactérienne comprenant des peptides bactéricides et bactériostatiques choisis dans le groupe comprenant les cécropines, les Penalida, les bacténécines, les callinectines, les myticines, les tachyplésines, les clavanines, les misgurines, les pleurocidines, les parasines, les histones, les protéines acides et les lysozymes non natifs vis-à-vis de ladite biomasse bactérienne.

7. Procédé selon la revendication 6, dans lequel la bactérie est Lactobacillus.

8. Procédé selon la revendication 7, dans lequel la bactérie probiotique est choisie dans le groupe comprenant Lactobacillus, Bacillus et Bifidobacterium.

9. Procédé selon la revendication 1, dans lequel l'étape c) correspond à une lyophilisation, un séchage par pulvérisation, un séchage en lit fluidisé, une microencapsulation, une extrusion ou une mise en comprimés.

10. Utilisation en aquaculture d'une composition nutritive pouvant être obtenue par le procédé selon la revendication 5 pour la préparation d'un médicament qui inhibe l'infection par le virus du syndrome de Taura (TSV) ou par le virus du syndrome des taches blanches (WSSV) chez la crevette.

11. Utilisation en aquaculture d'une composition nutritive pouvant être obtenue par le procédé selon la revendication 6 pour la préparation d'un médicament qui transmet une résistance à l'infestation.
